(19) Europäisches Patentamt
European Patent Office
Office européen des brevets

(11) Veröffentlichungsnummer: **0 123 187**
**B1**

(12) **EUROPÄISCHE PATENTSCHRIFT**

(45) Veröffentlichungstag der Patentschrift: **20.04.88**

(21) Anmeldenummer: **84103868.0**

(22) Anmeldetag: **07.04.84**

(51) Int. Cl.⁴: **C 07 C 67/00**, C 07 C 69/65, C 07 C 69/734, C 07 C 149/40, C 07 D 317/46, C 07 C 43/225, C 07 C 25/13, C 07 C 21/24, C 07 C 149/34, C 07 D 319/20

# (54) Verfahren zur Herstellung von fluorierten Phenylessigsäureestern und neue fluorierte Trichlorethylbenzole.

(30) Priorität: **20.04.83 DE 3314249**

(43) Veröffentlichungstag der Anmeldung: **31.10.84 Patentblatt 84/44**

(45) Bekanntmachung des Hinweises auf die Patenterteilung: **20.04.88 Patentblatt 88/16**

(84) Benannte Vertragsstaaten: **CH DE FR GB IT LI**

(56) Entgegenhaltungen:
**EP - A - 0 098 058**
**BE - A - 760 968**
**GB - A - 2 027 699**

(73) Patentinhaber: **BAYER AG, Konzernverwaltung RP Patentabteilung, D-5090 Leverkusen 1 Bayerwerk (DE)**

(72) Erfinder: **Marhold, Albrecht, Dr., Carl-Duisberg-Strasse 329, D-5090 Leverkusen 1 (DE)**

**EP 0 123 187 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von am aromatischen Kern und/oder in einer Seitenkette fluorierten Phenylessigsäureestern aus am aromatischen Kern und/oder in einer Seitenkette fluorierten β,β,β-Trichlorethylbenzolen und neue fluorierte Trichlorethylbenzole.

Es ist bekannt, dass man 3-Trifluormethylphenylessigsäuremethyl- und -ethylester in einem mehrstufigen Verfahren aus 3-Trifluormethylbenzotrichlorid herstellen kann (s. US-PS 4 268 687). Nachteilig bei diesem Verfahren ist nicht nur der 6 Stufen umfassende Syntheseweg, sondern auch das benötigte Ausgangsmaterial, das nur schwierig zugänglich ist und eine aufwendige Technologie voraussetzt.

Nicht-fluorierte Phenylessigsäureester können durch die Verseifung von Trichlorethylbenzolen hergestellt werden, die aus einer Meerwein-Arylierung von Vinylidenchlorid erhalten worden sind. Hierbei erhält man zunächst Phenylessigsäuren, die in einem weiteren Reaktionsschritt noch verestert werden müssen. Hierbei ist nachteilig, dass die Verseifung der Trichlorethylbenzole mit Schwefelsäure bei Bedingungen durchzuführen ist, welche die Bildung von Nebenreaktionen fördern und damit die Ausbeute an Phenylessigsäure stark erniedrigen (s. V.M. Naidan, A.V. Dumbrovskii, Zh. Obshch. Khim, Vol. 34, 1474, engl.). Dieses Verfahren eignet sich nicht für die Herstellung fluorierter Phenylessigsäuren bzw. fluorierter Phenylessigsäureester, da unter den Reaktionsbedingungen z.B. aus 3-β,β,β-Trichlorethyl-benzotrifluorid nur Homoisophthalsäure entsteht.

3-Trifluormethyl-β,β,β-trichlorethylbenzol kann aus Vinylidenchlorid und dem Diazoniumsalz des 3-Trifluormethylanilins hergestellt werden (s. DE-AS 2 016 809, Beispiel 124).

Die EP-A 98 058, bei der es sich um eine prioritätsältere, jedoch nicht vorveröffentlichte Anmeldung handelt, betrifft ein Verfahren zur Herstellung bestimmter Phenylacetate durch Hydrolyse der entsprechenden Trichlorverbindungen. Das Verfahren und die Stoffe der vorliegenden Erfindung sind durch die EP-A 98 058 nicht vorweggenommen.

In der BE-A 760 968 wird die Herstellung von Phenylessigsäuren und Phenylessigsäureestern beschrieben. Phenylessigsäuren werden erhalten, indem man β,β,β-Trichlorethylbenzole mit wässriger KOH oder NaOH in Gegenwart eines Glykols erhitzt und aus dem dabei gebildeten Phenylacetat mit verdünnter Schwefelsäure die entsprechende Phenylessigsäure freisetzt. Die vorliegende Erfindung liefert mit formal ähnlich erscheinenden Massnahmen ein anderes Produkt, nämlich Phenylessigsäureester.

Phenylessigsäureester sind gemäss der BE-A 760 968 aus Phenylessigsäuren durch Umsetzung mit Thionylchlorid zum entsprechenden Phenylessigsäurechlorid und dessen Umsetzung mit Alkohol erhältlich. Es handelt sich hierbei um ein kompliziertes, mehrstufiges Verfahren.

Es wurde nun ein Verfahren zur Herstellung von am aromatischen Kern und/oder in einer Seitenkette fluorierten Phenylessigsäureestern gefunden, das dadurch gekennzeichnet ist, dass man fluorierte β,β,β-Trichlorethylbenzole der Formel

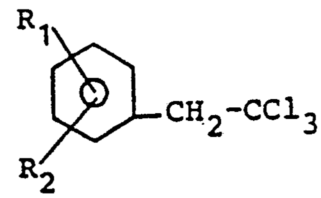

in der

$R_1$ für F oder eine der Gruppen $-OCF_3$, $-OCHF_2$, $-SCF_3$, $-SCHF_2$, $-CH_2-CF_3$, $-CH_2CH_2CF_3$, $-CF(CF_3)_2$, $-OCF_2-CHFCl$, $-SCF_2-CHFCl$, $-OCF_2-CF_2H$ oder $-SCF_2-CF_2H$ und

$R_2$ für Wasserstoff, Halogen, Alkyl, Alkoxy, Nitro oder für eine der bei $R_1$ angegebenen Gruppen steht, oder

$R_1$ und $R_2$ gemeinsam für eine der Gruppen $-O-CF_2-O-$, $-O-CF_2-CF_2-O-$, $-O-CF_2-CHF-O$, $-O-CF_2-CFCl-O-$, $-O-CF_2-CH_2-O-$ oder $-O-CF_2-O-CF_2-$

stehen, mit einem Alkoholat und anschliessend mit einer Säure umsetzt. Soweit dabei $R_2$ für Halogen steht bedeutet es vorzugsweise Fluor, Chlor oder Brom, besonders bevorzugt Fluor oder Chlor. Soweit dabei $R_2$ für Alkyl steht bedeutet es vorzugsweise $C_1$-$C_4$-Alkyl und soweit dabei $R_2$ für Alkoxy steht bedeutet es vorzugsweise $C_1$-$C_4$-Alkoxy.

Vorzugsweise steht $R_1$ für eine der Gruppen $-OCF_3$, $-OCHF_2$, $-SCF_3$, $-SCHF_2$, $-CH_2-CF_3$, $-OCF_2-CHFCl$, $-OCF_2-CF_2H$ oder $-SCF_2-CF_2H$.

Vorzugsweise steht $R_2$ für Wasserstoff, Fluor, Chlor, Methyl, Ethyl, Methoxy, Ethoxy oder für eine der bei $R_1$ als bevorzugt angegebenen Gruppen.

Für den Fall, dass $R_1$ und $R_2$ gemeinsam für eine zweibindige Gruppe stehen, steht $R_1$ und $R_2$ gemeinsam vorzugsweise für eine der Gruppen $-O-CF_2-O-$, $-O-CF_2-CF_2-O-$, $-O-CF_2-CHF-O-$, $-O-CF_2-CFCl-O-$ oder $O-CF_2-O-CF_2-$.

Besonders bevorzugt werden in das erfindungsgemässe Verfahren folgende fluorierte β,β,β-Trichlorethylbenzole eingesetzt:

| Substituent(en) | | Position(en) | |
|---|---|---|---|
| $R_1$ | $R_2$ | | |
| $CF_3O$ | | 4 | |
| $CF_3O$ | | 2 | |
| $CF_3S$ | | 4 | |
| $-O-CF_2-O-$ | | 3 | 4 |
| $-O-CF_2-CHF-O-$ | | 3 | 4 |
| $-O-CF_2-CF_2-O-$ | | 3 | 4 |

Bei nur einer Eintragung steht $R_2$ für H

Als Alkoholate für die erfindungsgemässe Umsetzung können die verschiedensten aliphatischen oder aromatischen Alkoholate eingesetzt werden. Vorzugsweise werden Alkoholate eingesetzt, die sich von aliphatischen Alkoholen mit 1 bis 4 C-Atomen ableiten und als Gegenion zum Alkoholatrest Alkali- und/oder Erdalkaliionen enthalten. Besonders bevorzugt ist der Einsatz von Natriummethylat.

Prinzipiell kann das Alkoholat in beliebiger Menge eingesetzt werden. Bevorzugt ist der Einsatz eines molaren Überschusses an Alkoholat, also der Einsatz von mehr als 3 Mol Alkoholat, bezogen auf 1 Mol fluoriertes β,β,β-Trichlorethylbenzol. Besonders bevorzugt werden pro Mol fluoriertes β,β,β-Trichlorethylbenzol 3,1 bis 5 Mol Alkoholat eingesetzt.

Die Umsetzung mit dem Alkoholat wird im allgemeinen in Gegenwart von Lösungsmitteln durchgeführt. Geeignete Lösungsmittel sind beispielsweise aprotische Lösungsmittel wie Ether, z.B. Diethylether, Glykoldimethylether und Dioxan; Sulfone, z.B. Tetramethylensulfon; und Carbonsäureamide, z.B. Dimethylformamid und Diethylacetamid. Bevorzugte Lösungsmittel sind Alkohole, gegebenenfalls in Kombination mit einem der vorgenannten aprotischen Lösungsmittel. Besonders bevorzugt verwendet man als Lösungsmittel den dem eingesetzten Alkoholat entsprechenden Alkohol, also z.B. im Falle des Einsatzes von Natriummethylat Methanol. Das oder die Lösungsmittel können der Umsetzung als solche zugefügt werden. Bevorzugt setzt man eine alkoholische Alkoholatlösung zu und sonst kein weiteres Lösungsmittel.

Die Menge des Lösungsmittels kann in weiten Grenzen variiert werden, vorzugsweise wird mindestens soviel Lösungsmittel eingesetzt, dass ein gut rührbares Reaktionsgemisch entsteht.

Die Umsetzung mit dem Alkoholat kann beispielsweise bei Temperaturen im Bereich von 20 bis 120°C vorgenommen werden. Diese Umsetzung wird im allgemeinen bei Normaldruck durchgeführt, sie kann jedoch auch bei vermindertem oder erhöhtem Druck durchgeführt werden.

Eine Isolierung der bei der Umsetzung der fluorierten β,β,β-Trichlorethylbenzole mit den Alkoholaten entstehenden Umsetzungsprodukte ist nicht erforderlich, kann aber gegebenenfalls durchgeführt werden, beispiels- durch Destillation. Die Produkte aus der Umsetzung der fluorierten β,β,β-Trichlorethylbenzole mit den Alkoholaten, vorzugsweise jedoch das gesamte bei der Umsetzung der fluorierten β,β,β-Trichlorethylbenzole mit den Alkoholaten entstehende Reaktionsgemisch wird erfindungsgemäss mit einer Säure umgesetzt. Im allgemeinen wird dabei soviel Säure zugesetzt, dass ein pH-Wert von kleiner als 5 resultiert. Bevorzugt wird mit der Säure ein pH-Wert im Bereich von 4,5 bis 1 eingestellt.

Als Säuren sind die verschiedensten Säuren geeignet, mit denen sich die angegebenen pH-Werte erreichen lassen. Vorzugsweise werden starke anorganische Säuren verwendet, beispielsweise Salzsäure oder Schwefelsäure. Besonders bevorzugt wird Schwefelsäure verwendet.

Die Umsetzung mit der Säure kann beispielsweise bei Temperaturen im Bereich von 20 bis 120°C erfolgen. Im allgemeinen verfährt man dabei so, dass man die Säure zunächst bei relativ niedriger Temperatur, z.B. im Bereich 20 bis 50°C, und gegebenenfalls unter Kühlung zugibt und die Umsetzung mit der Säure bei höheren Temperaturen, z.B. im Bereich 50 bis 120°C, zu Ende führt.

Die Isolierung der nach der Umsetzung mit der Säure im Reaktionsgemisch vorhandenen fluorierten Phenylessigsäureester kann auf verschiedene Weise erfolgen, beispielsweise durch Extraktion oder Destillation oder eine Kombination von Destillations- und Extraktionsverfahren.

Bei einer bevorzugten Aufarbeitung von Reaktionsgemischen, die ohne Zwischenisolierung nach der Umsetzung mit Alkoholat nach der Umsetzung mit Säure erhalten worden sind, wird wie folgt verfahren:

Zunächst werden der dem eingesetzten Alkoholat entsprechende Alkohol und gegebenenfalls, der als Lösungsmittel eingesetzte Alkohol und gegebenenfalls vorhandene sonstige Lösungsmittel durch Destillation, gegebenenfalls unter vermindertem Druck, abgetrennt. Das dabei verbleibende Sumpfprodukt wird mit mindestens soviel Wasser versetzt, dass sich zwei klare Phasen bilden. Die wässrige Phase wird dann verworfen und die organische Phase mit einem organischen Lösungsmittel extrahiert. Hierfür geeignete Lösungsmittel sind beispielsweise chlorierte Kohlenwasserstoffe, insbesondere Dichlormethan. Aus dem so erhaltenen Extrakt wird dann das Lösungsmittel durch Destillation, gegebenenfalls unter vermindertem Druck, entfernt und der hinterbleibende Rückstand im Vakuum, beispielsweise bei Drücken im Bereich von 5 bis 50 mbar, destilliert.

Nach dieser oder einer anderen Aufarbeitung der Reaktionsgemische erhält man die dem eingesetzten fluorierten β,β,β-Trichlorethylbenzol und dem eingesetzten Alkoholat entsprechenden fluorierten Phenylessigsäureester. Falls ein nicht dem eingesetzten Alkoholat entsprechender Alkohol als Lösungsmittel eingesetzt worden ist, können Gemische verschiedener fluorierter Phenylessigsäureester entstehen.

Es ist ausgesprochen überraschend, dass es erfindungsgemäss gelingt, im aromatischen Kern und/oder in einer Seitenkette fluorierte Phenylessigsäureester in guten Ausbeuten zu erhalten, da zu erwarten war, dass während des erfindungsgemässen Verfahrens eine Verseifung der fluorhaltigen Substituenten eintritt.

Die vorliegende Erfindung betrifft weiterhin neue fluorierte β,β,β-Trichlorethylbenzole der Formel

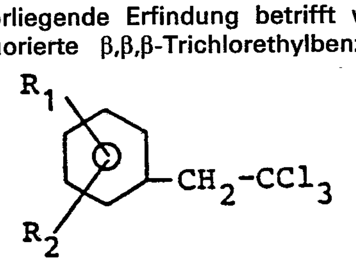

in der

$R_1$ für F oder eine der Gruppen $-OCF_3$, $-SCF_3$, $-CH_2CF_3$, $-CH_2-CH_2CF_3$, $-CF(CF_3)_2$, $-OCF_2-CHFCl$, $-SCF_2-CHFCl$, $-OCF_2-CF_2H$ oder $-SCF_2-CF_2H$ und

$R_2$ für Wasserstoff, Halogen, Alkyl, Alkoxy, Nitro oder für eine der bei $R_1$ angegebenen Gruppen steht, oder

$R_1$ und $R_2$ gemeinsam für eine der Gruppen $-O-CF_2-O-$, $-O-CF_2-CF_2-O-$, $-O-CF_2-CHF-O-$, $-O-CF_2-CFCl-O-$, $-O-CF_2-CH_2-O-$ oder $-O-CF_2-O-CF_2-$

stehen.

Soweit dabei $R_2$ für Halogen steht bedeutet es vorzugsweise Fluor, Chlor oder Brom, besonders bevorzugt Fluor oder Chlor. Soweit dabei $R_2$ für Alkyl steht bedeutet es vorzugsweise $C_1$-$C_4$-Alkyl und soweit dabei $R_2$ für Alkoxy steht bedeutet es vorzugsweise $C_1$-$C_4$-Alkoxy.

Vorzugsweise steht $R_1$ für eine der Gruppen $-OCF_3$, $-SCF_3$, $-CH_2-CF_3$, $-OCF_2-CHFCl$, $-OCF_2-CF_2H$ oder $-SCF_2-CF_2H$.

Vorzugsweise steht $R_2$ für Wasserstoff, Fluor, Chlor, Methyl, Ethyl, Methoxy, Ethoxy oder für eine der bei $R_1$ als bevorzugt angegebenen Gruppen.

Für den Fall, dass $R_1$ und $R_2$ gemeinsam für eine zweibindige Gruppe stehen, steht $R_1$ und $R_2$ gemeinsam vorzugsweise für eine der Gruppen $-O-CF_2-O-$, $-O-CF_2-CF_2-O-$, $-O-CF_2-CHF-O-$, $-O-CF_2CFCl-O-$ oder $-O-CF_2-O-CF_2-$.

Besonders bevorzugte fluorierte β,β,β-Trichlorethylbenzole sind die folgenden:

| Substituent(en) $R_1$ | $R_2$ | Position(en) | |
|---|---|---|---|
| $CF_3O$ | | 4 | |
| $CF_3O$ | | 2 | |
| $CF_3S$ | | 4 | |
| $-O-CF_2-O-$ | | 3 | 4 |
| $O-CF_2-CHF-O-$ | | 3 | 4 |
| $O-CF_2-CF_2-O-$ | | 3 | 4 |
| $O-CF_2CFCl-O-$ | | 3 | 4 |

Bei nur einer Eintragung steht $R_2$ für H

Es ist bekannt, dass man Aniline, z.B. 3-Trifluormethyl-anilin, durch Umsetzung mit Nitrit in Gegenwart von Salzsäure diazotieren und das so zugängliche Diazoniumsalz mit Vinylidenchlorid in Gegenwart von Aceton und Katalysatoren, z.B. Kupferchloriden, zu den entsprechenden β,β,β-Trichlorethylbenzolen, z.B. 3-Trifluormethyl-β,β,β-trichlorethylbenzol, umsetzen kann (s. beispielsweise J. f. prak. Chemie, 152, S. 237-266 (1939) und DE-AS 2 016 890).

In analoger Weise sind die neuen fluorierten β,β,β-Trichlorethylbenzole zugänglich, indem man Aniline der Formel

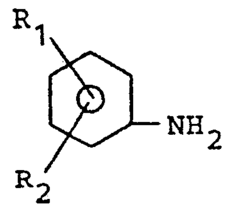

in der

$R_1$ und $R_2$ die oben angegebene Bedeutung haben, $R_2$ aber nicht für Wasserstoff steht, wenn $R_1$ für eine Trifluormethylgruppe in 3-Stellung zur $NH_2$-Gruppe steht,

diazotiert, z.B. mit einer äquimolaren Menge an Natriumnitrit in Gegenwart von Salzsäure und Wasser bei Temperaturen unter 5°C, und das so erhaltene Diazoniumsalz des eingesetzten Anilins mit Vinylidenchlorid in Gegenwart von Aceton und einem Kupferchlorid bei Temperaturen von beispielsweise 20 bis 80°C umsetzt.

Die neuen fluorierten β,β,β-Trichlorethylbenzole sind wertvolle Zwischenprodukte, weil man diese, wie zuvor beschrieben, auf einfache Weise und in guten Ausbeuten zu am aromatischen Kern und/oder in einer Seitenkette fluorierten Phenylessigsäureestern umsetzen kann, die ihrerseits wichtige Zwischenprodukte zur Herstellung von Herbiziden sind (s. z.B. DE-OS 2 537 753 und DE-OS 2 628 992).

Insgesamt betrachtet wird hiermit also ein besonders günstiger Weg zu am aromatischen Kern und/oder in einer Seitenkette fluorierten Phenylessigsäureestern eröffnet, bei dem ausgehend von gut zugänglichen, am aromatischen Kern und/oder in einer Seitenkette fluorierten Anilinen zunächst die entsprechenden β,β,β-Trichlorethylbenzole hergestellt werden, die mit Ausnahme des 3-Trifluormethyl-β,β,β-trichlorethylbenzols neu sind, und diese dann in die entsprechenden Phenylessigsäureester überführt.

Die folgenden Beispiele erläutern die vorliegende Erfindung.

Beispiele

Beispiel 1

1,5 Mol 4-Trifluormethoxyanilin wurden zusammen mit 700 ml Wasser und 450 ml 37%iger Salzsäure bei 0°C vorgelegt und durch Zutropfen einer Lösung von 103,5 g Natriumnitrit in 330 ml Wasser diazotiert. Durch Kühlung wurde die Temperatur bei -5 bis 0°C gehalten. Nach dem Ende der Zugabe der Natriumnitritlösung wurde 1 Stunde nachgerührt und Amidosulfonsäure zugegeben, um evtl. noch vorhandenes Nitrit zu zerstören. Die erhaltene, gekühlte Diazoniumlösung wurde durch ein Filter zu einer Mischung von 1200 ml Aceton, 450 g Vinylidenchlorid und 15 g Kupferchlorid fliessen gelassen. Es wurde bis zum Ende der Gasentwicklung bei 25 bis 30°C gerührt und anschliessend noch für 2 Stunden am Rückfluss gehalten. Nach dem Abdestillieren von überschüssigem Vinylidenchlorid und Aceton wurde die organische Phase im Sumpf abgetrennt und das so erhaltene rohe 4-Trifluormethoxy-β,β,β-trichlorethylbenzol durch Destillation über eine Kolonne gereinigt. Das so erhaltene 4-Trifluormethoxy-β,β,β-trichlorethylbenzol hatte einen Siedepunkt von 113-117°C bei 20 mbar, einen Brechungsindex von $n_D^{20}$ von 1,4790 und wurde in einer Ausbeute von 58% der Theorie erhalten.

Beispiele 2 bis 6

In entsprechender Weise wie in Beispiel 1 beschrieben wurden weitere fluorierte Aniline in die entsprechenden β,β,β-Trichlorethylbenzole überführt. Die eingesetzten Aniline, die erhaltenen β,β,β-Trichlorethylbenzole, die gemessenen physikalischen Daten der erhaltenen β,β,β-Trichlorethylbenzole und die Ausbeuten, in denen diese erhalten wurden sind in der folgenden Tabelle 1 zusammengefasst.

Tabelle 1

| Beispiel Nr. | eingesetztes Anilin | erhaltenes β,β,β-Trichlorethylbenzol | physikalische Daten | Ausbeute |
|---|---|---|---|---|
| 2 | 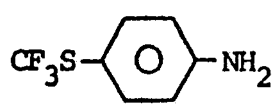 | 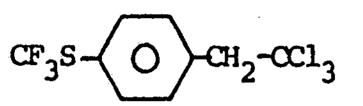 | Kp: 132–136°C bei 16 mbar Fp: 76–78°C | 88% |
| 3 | 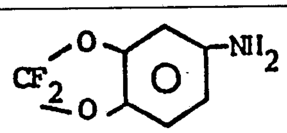 | 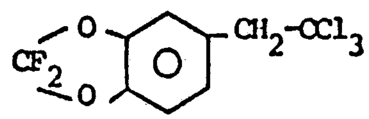 | Kp: 127–130°C bei 16 mbar $n_D^{20}$: 1,5096 | 56% |
| 4 | 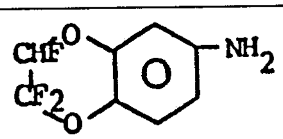 | 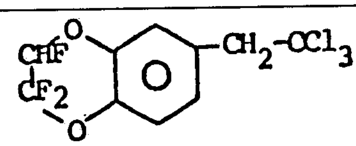 | Kp: 150–153°C bei 16 mbar $n_D^{20}$: 1,5074 | 48% |
| 5 | 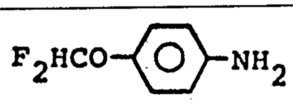 | 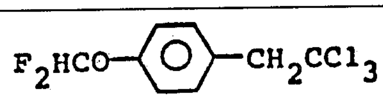 | Kp: 124–125°C bei 18 mbar | 68% |
| 6 | 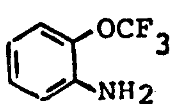 | 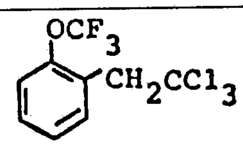 | Kp: 110–112°C bei 18 mbar $n_D^{20}$: 1,4823 | |

Beispiel 7

Zu 0,27 Mol 4-Trifluormethoxy-β,β,β-trichlorethylbenzol, das in 100 ml Methanol vorgelegt wurde, wurden 135 ml einer 30%igen Natriummethylatlösung in Methanol bei Raumtemperatur zugetropft. Nach dem Ende der Zugabe wurde 3 Stunden lang am Rückfluss gekocht, danach auf 20°C abgekühlt, 30 ml konzentrierte Schwefelsäure zugegeben, 1 Stunde lang am Rückfluss gekocht und anschliessend das Methanol abdestilliert. Das zurückbleibende Produkt wurde mit soviel Wasser versetzt, dass sich zwei klare Phasen bildeten, die organische Phase wurde mit Dichlormethan extrahiert, das Dichlormethan abdestilliert und anschliessend 4-Trifluormethoxyphenylessigsäuremethylester durch Destillation im Vakuum in einer Ausbeute von 83% erhalten. Der Siedepunkt des 4-Trifluormethoxyphenylessigsäuremethylesters lag bei 107°C/20 mbar sein Brechungsindex $n_D^{20}$ betrug 1,4445.

Beispiele 8 bis 12

In entsprechender Weise wie in Beispiel 7 beschrieben wurden weitere fluorierte β,β,β-Trichlorethylbenzole in die entsprechenden fluorierten Phenylessigsäuremethylester überführt. Die eingesetzten fluorierten β,β,β-Trichlorethylbenzole, die erhaltenen fluorierten Phenylessigsäuremethylester, die gemessenen physikalischen Daten der erhaltenen fluorierten Phenylessigsäuremethylester und die Ausbeuten, in denen diese erhalten wurden sind in der folgenden Tabelle 2 zusammengefasst.

Tabelle 2

| Beispiel Nr. | eingesetztes β,β,β-Trichlorethylbenzol $X = -CH_2-CCl_3$ | erhaltener Phenyl. essigsäuremethylester $Y = -CH_2-CO_2-CH_3$ | physikalische Daten | Ausbeute |
|---|---|---|---|---|
| 8 | 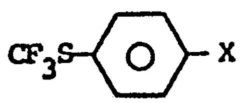 | 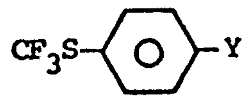 | Kp: 123–124°C bei 16 mbar $n_D^{20}$ : 1,4849 | 82% |
| 9 | 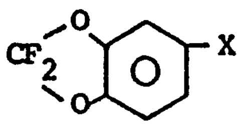 | 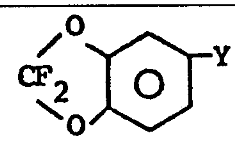 | Kp: 120–123°C bei 16 mbar $n_D^{20}$ : 1,4730 | 58% |
| 10 | 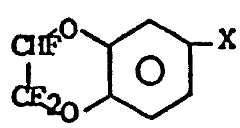 | 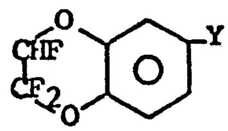 | Kp: 145–146°C bei 20 mbar $n_D^{20}$ : 1,4770 | 81% |
| 11 | 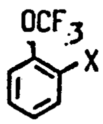 | 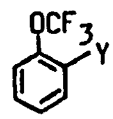 | Kp: 104–105°C bei 16 mbar | 79% |
| 12 | 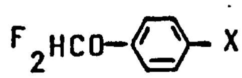 | 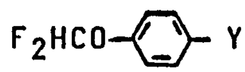 | Kp: 106–108°C bei 10 mbar | 82% |

**Patentansprüche**

1. Verfahren zur Herstellung von am aromatischen Kern und/oder in einer Seitenkette fluorierten Phenylessigsäureestern, dadurch gekennzeichnet, dass man fluorierte β,β,β-Trichlorethylbenzole der Formel

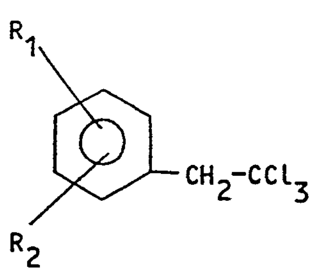

,

in der

$R_1$ für eine der Gruppen $-OCF_3$, $-OCHF_2$, $-SCF_3$, $-SCHF_2$, $-CH_2-CF_3$, $-CH_2-CH_2-CF_3$, $-CF(CF_3)_2$, $OCF_2-CHFCl$, $-SCF_2-CHFCl$, $-OCF_2-CF_2H$ oder $-SCF_2-CF_2H$ und

$R_2$ für Wasserstoff, Halogen, Alkyl, Alkoxy, Nitro oder für eine der bei $R_1$ angegebenen Gruppen steht, oder

$R_1$ und $R_2$ gemeinsam für eine der Gruppen $-O-CF_2-O-$, $-O-CF_2-CF_2-O-$, $-O-CF_2-CHF-O-$, $-O-CF_2-CFCl-O-$, $-O-CF_2-CH_2-O-$ oder $-O-CF_2-O-CF_2-$

stehen,

mit einem Alkoholat und anschliessend mit einer Säure umsetzt.

2. Verfahren nach Anspruch 1, dadurch gekennzeichnet, dass man, bezogen auf 1 Mol fluoriertes β,β,β-Trichlorethylbenzol, mindestens 3 Mol eines Alkoholats einsetzt.

3. Verfahren nach Ansprüchen 1 bis 2, dadurch gekennzeichnet, dass man ein Alkali- und/oder Erdalkalialkoholat eines aliphatischen Alkohols mit 1 bis 4 C-Atomen einsetzt.

4. Verfahren nach Ansprüchen 1 bis 3, dadurch gekennzeichnet, dass man die Umsetzung mit dem Alkoholat in Gegenwart eines aprotischen Lösungsmittels und/ oder in Gegenwart eines Alkohols durchführt.

5. Verfahren nach Ansprüchen 1 bis 4, dadurch gekennzeichnet, dass man die Umsetzung mit dem Alkoholat bei Temperaturen im Bereich von 20 bis 120°C durchführt.

6. Verfahren nach Ansprüchen 1 bis 5, dadurch gekennzeichnet, dass man zur Umsetzung mit einer Säure soviel Säure zusetzt, dass ein pH-Wert von kleiner als 5 resultiert.

7. Verfahren nach Ansprüchen 1 bis 6, dadurch gekennzeichnet, dass man die Umsetzung mit einer Säure mit einer starken anorganischen Säure vornimmt.

8. Verfahren nach Ansprüchen 1 bis 7, dadurch gekennzeichnet, dass man die Umsetzung mit einer Säure bei Temperaturen im Bereich von 20 bis 120°C durchführt.

9. Fluorierte β,β,β-Trichlorethylbenzole der Formel

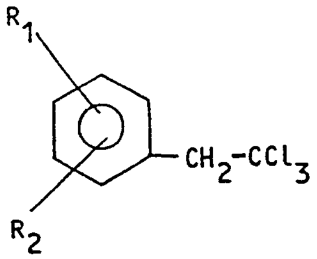

in der

$R_1$ für eine der Gruppen $-OCF_3$, $-SCF_3$, $-CH_2-CF_3$, $-CH_2-CH_2CF_3$, $-CF(CF_3)_2$, $-OCF_2-CHFCl$, $-SCF_2-CHFCl$, $-OCF_2-CF_2H$ oder $-SCF_2-CF_2H$ und

$R_2$ für Wasserstoff, Halogen, Alkyl, Alkoxy, Nitro oder für eine der bei $R_1$ angegebenen Gruppen steht, oder

$R_1$ und $R_2$ gemeinsam für eine der Gruppen $-O-CF_2-O-$, $-O-CF_2-CF_2-O-$, $-O-CF_2-CHF-O-$, $-O-CF_2-CFCl-O-$, $-O-CF_2-CH_2-O-$ oder $-O-CF_2-O-CF_2$ stehen.

**Claims**

1. Process for the preparation of phenylacetates which are fluorinated at the aromatic nucleus and/or in a side-chain, characterized in that fluorinated β,β,β-trichloroethylbenzenes of the formula

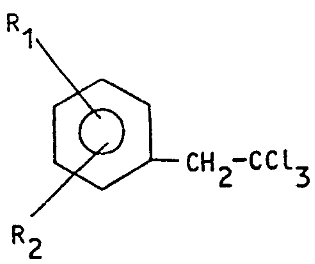

in which

$R_1$ represents one of the groups $-OCF_3$, $-OCHF_2$, $-SCF_3$, $-SCHF_2$, $-CH_2-CF_3$, $CH_2-CH_2-CH_3$, $-CF(CF_3)_2$, $OCF_2-CHFCl$, $-SCF_2-CHFCl$, $-OCF_2-CF_2-H$ or $-SCF_2-CF_2H$ and

$R_2$ represents hydrogen, halogen, alkyl, alkoxy, nitro or one of the groups stated for $R_1$, or

$R_1$ and $R_2$ together represent one of the groups $-O-CF_2-O-$, $-O-CF_2-CF_2-O-$, $-O-CF_2-CHF-O-$, $-O-CF_2-CFCl-O-$, $-O-CF_2-CH_2-O-$ or $-O-CF_2-O-CF_2-$ are reacted with an alcoholate and then with an acid.

2. Process according to Claim 1, characterized in that at least 3 mol of an alcoholate are employed per mol of fluorinated β,β,β-trichloroethylbenzene.

3. Process according to Claims 1 or 2, characterized in that an alkali metal and/or alkaline earth metal alcoholate of an aliphatic alcohol having 1 to 4 carbon atoms is employed.

4. Process according to Claims 1 to 3, characterized in that the reaction with the alcoholate is carried out in the presence of an aprotic solvent and/or in the presence of an alcohol.

5. Process according to Claims 1 to 4, characterized in that the reaction with the alcoholate is carried out at temperatures in the range from 20 to 120°C.

6. Process according to Claims 1 to 5, characterized in that, for the reaction with an acid, the amount of acid added is such that a pH value of less than 5 results.

7. Process according to Claims 1 to 6, characterized in that the reaction with an acid is carried out using a strong inorganic acid.

8. Process according to Claims 1 to 7, characterized in that the reaction with an acid is carried out at temperatures in the range from 20 to 120°C.

9. Fluorinated β,β,β-trichloroethylbenzenes of the formula

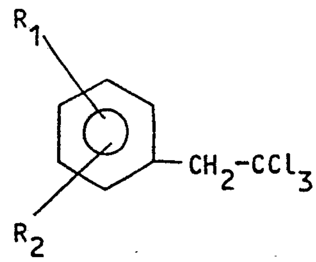

in which

$R_1$ represents one of the groups $-OCF_3$, $-SCF_3$, $-CH_2-CF_3$, $CH_2-CH_2-CF_3$, $-CF(CF_3)_2$, $-OCF_2-CHFCl$, $-SCF_2-CHFCl$, $-OCF_2-CF_2H$ or $-SCF_2-CF_2H$ and

$R_2$ represents hydrogen, halogen, alkyl, alkoxy, nitro or one of the groups stated for $R_1$, or

$R_1$ and $R_2$ together represent one of the groups $-O-CF_2-O-$, $O-CF_2-CF_2-O-$, $O-CF_2-CHF-O-$, $-O-CF_2-CFCl-O-$, $-O-CF_2-CH_2-O-$ or $-O-CF_2-O-CF_2-$.

**Revendications**

1. Procédé de préparation d'esters phénylacétiques fluorés sur le noyau aromatique et/ou dans une chaîne latérale, caractérisé en ce que l'on fait réagir des β,β,β-trichloréthyl-benzènes fluorés de formule

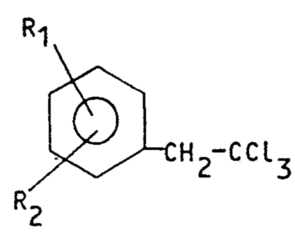

dans laquelle

$R_1$ représente l'un des groupes $-OCF_3$, $-OCHF_2$, $-SCF_3$, $-SCHF_2$, $-CH_2CF_3$, $-CH_2CH_2-CF_3$, $-CF(CF_3)_2$, $-OCF_2-CHFCl$, $-SCF_2-CHFCl$, $-OCF_2CF_2H$ ou $-SCF_2CF_2H$, et

$R_2$ représente l'hydrogène, un halogène, un groupe alkyle, alcoxy, nitro ou l'un des groupes indiqués en référence à $R_1$, ou bien

$R_1$ et $R_2$ forment ensemble l'un des groupes $-O-CF_2-O-$, $-O-CF_2-CF_2-O-$, $-O-CF_2-CHF-O-$, $-O-$

$CF_2$-CFCl-O-, -O-$CF_2$-$CH_2$-O- ou -O-$CF_2$-O-$CF_2$, avec un alcoolate puis avec un acide.

2. Procédé selon la revendication 1, caractérisé en ce que l'on utilise au moins 3 moles d'un alcoolate par mole de β,β,β-trichloréthylbenzène fluoré.

3. Procédé selon les revendications 1 et 2, caractérisé en ce que l'on utilise un alcoolate alcalin et/ou alcalinoterreux d'un alcool aliphatique en $C_1$-$C_4$.

4. Procédé selon les revendications 1 à 3, caractérisé en ce que la réaction avec l'alcoolate est effectuée en présence d'un solvant aprotonique et/ou en présence d'un alcool.

5. Procédé selon les revendications 1 à 4, caractérisé en ce que la réaction avec l'alcoolate est effectuée à des températures dans l'intervalle de 20 à 120°C.

6. Procédé selon les revendications 1 à 5, caractérisé en ce que, pour la réaction avec un acide, on ajoute l'acide en quantité suffisante pour parvenir à un pH inférieur à 5.

7. Procédé selon les revendications 1 à 6, caractérisé en ce que la réaction avec un acide est effectuée avec un acide minéral fort.

8. Procédé selon les revendications 1 à 7, caractérisé en ce que la réaction avec un acide est effectuée à des températures dans l'intervalle de 20 à 120°C.

9. β,β,β-trichloroéthylbenzènes fluorés de formule

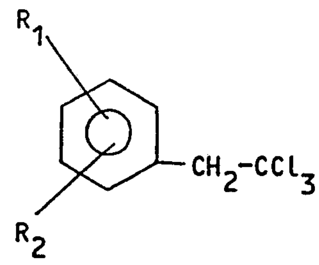

dans laquelle

$R_1$ représente l'un des groupes -$OCF_3$, -$SCF_3$, -$CH_2$-$CF_3$, -$CH_2$-$CH_2$-$CF_3$, -$CF(CF_3)_2$, -$OCF_2$-CHFCl, -$SCF_2$-CHFCl, -$OCF_2$-$CF_2$H ou -$SCF_2$-$CF_2$H, et

$R_2$ représente l'hydrogène, un halogène, un groupe alkyle, alcoxy, nitro ou l'un des groupes indiqués en référence à $R_1$, ou bien

$R_1$ et $R_2$ forment ensemble l'un des groupes -O-$CF_2$-O-, -O-$CF_2$-$CF_2$-O-, -O-$CF_2$-CHF-O-, -O-$CF_2$-CFCl-O-, -O-$CF_2$-$CH_2$-O- ou -O-$CF_2$-O-$CF_2$-.